# EUROPÄISCHE PATENTSCHRIFT

(11) **EP 2 559 751 B1**
(45) Veröffentlichungstag und Bekanntmachung des Hinweises auf die Patenterteilung: **18.05.2016**
(21) Anmeldenummer: 12180162.5
(22) Anmeldetag: 10.08.2012
(51) Int. Cl.: C12M 1/00, C12M 1/107, C12M 1/18, C12M 1/26, C12M 1/34

(54) **Behälter zur Vorbehandlung organischer Stoffe**
Container for the pre-treatment of organic substances
Récipient destiné au prétraitement de matières organiques

(30) Priorität: 19.08.2011 DE 102011081286
(43) Veröffentlichungstag der Anmeldung: 20.02.2013
(73) Patentinhaber: Rohn, Peter, 91610 Insingen (DE)
(72) Erfinder: Rohn, Peter, 91610 Insingen (DE)
(74) Vertreter: DREISS Patentanwälte PartG mbB

(56) Entgegenhaltungen:
- EP-A1- 0 530 797
- EP-A2- 2 177 280
- WO-A1-2006/089766
- DE-A1- 3 610 422
- DE-A1-102006 047 828
- DE-B- 1 063 516
- DE-C1- 19 621 914
- DE-U1-202009 015 199
- GB-A- 621 772

## Beschreibung

Die Erfindung trifft einen Behälter zur Vorbehandlung organischer Stoffe, angeordnet in einer Anlage zur Erzeugung von Biogas.

In dem Behälter werden feste organische Stoffe mit Gülle oder flüssigen Gärresten zu einem Substrat gemischt, welches dann per Entnahmepumpe in regelmäßigen Abständen in einen Fermenter gefördert wird. Bei kleinen Behältern, sogenannten Anmaischbehältern, wird fast der gesamte Inhalt gleich abgepumpt. Eine Befüllung mit festen Stoffen erfolgt durch Radlader oder kann auch per Förderband aus einem Dosierbehälter erfolgen. Zusätzlich werden die Gerüche und Gase schon im Behälter durch ein permanent laufendes Gebläse stark verdünnt und zumeist über einen Biofilter abgesaugt. Grundsätzlich sind die Belange des Explosionsschutzes vom für die Anlage zuständigen Sachverständigen für Betriebssicherheit zu prüfen.

Bei der Befüllung steht eine 1m x 2m messende Einwurföffnung längere Zeit, ca. 0,5 bis 2 Stunden pro Tag offen. Da das Substrat bei der Einfüllung gerührt werden muss, tritt hier eine starke Geruchsbelästigung auf.

Eine Alternative zur Einwurföffnung stellen stationäre Feststoffdosierer mit mehreren Misch- und Förderschnecken dar, die aber einem hohen Verschleiß unterliegen und einen hohen Energiebedarf haben. Außerdem liegt die Einbringleistung der Förderschnecken bei nur rund einem Viertel derjenigen die bei einem Direkteinwurf mittels Radlader erzielt werden kann.

In der EP 2 177 280 A2 wird funktionell und ohne Ausführungsbeispiel eine Schleuse zwischen einem Druckbehälter und einem Hydrolizer beschrieben.

Die WO 2006/89766 A1 beschreibt einen Hydrolysereaktor, ohne zu beschreiben, wie das Frischgut in den Hydrolysereaktor eingebracht wird.

Aus der DE 10 2006 047 828 A1 ist ein Fermenter bekannt, der zwei Schleusen aufweist. Diese Schleusen werden entweder durch einen aufblasbaren Formkörper oder Perkolat ausgefüllt, um das in den Schleusen befindliche Biogas wieder zurück in den Fermenter zu drücken.

Aus der DE 20 2009 015 199 U1 ist ein schleusenartiger mit Flüssigkeit gefüllter Zwischenbehälter bekannt.

Das der Erfindung zugrunde liegende Problem wird durch einen Behälter nach Anspruch 1 gelöst. Vorteilhafte Weiterbildungen sind in Unteransprüchen angegeben. Für die Erfindung wichtige Merkmale finden sich ferner in der nachfolgenden Beschreibung und in den Zeichnungen, wobei die Merkmale sowohl in Alleinstellung als auch in unterschiedlichen Kombinationen für die Erfindung wichtig sein können, ohne dass hierauf nochmals explizit hingewiesen wird.

Durch die Zuführung der organischen Feststoffe über ein Schleusensystem wird die Geruchsbelästigung gegenüber herkömmlichen, während der Zuführung permanent geöffneter Systeme deutlich reduziert.

Außerdem wird gegenüber der Verwendung von Schneckenförderern der Energie und Wartungsaufwand erheblich reduziert.

Nachfolgend werden beispielhafte Ausführungsformen der Erfindung unter Bezugnahme auf die Zeichnung erläutert. In der Zeichnung zeigen:
- Figur 1: das Umfeld der Erfindung
- Figur 2: eine Vorderansicht der Erfindung
- Figur 3: eine Seitenansicht der Erfindung
- Figur 4: eine Draufsicht der Erfindung
- Figur 5: eine erste Detailansicht einer Rinne
- Figur 6: eine zweite Detailansicht einer Rinne
- Figur 7: ein Anmaischbehälter

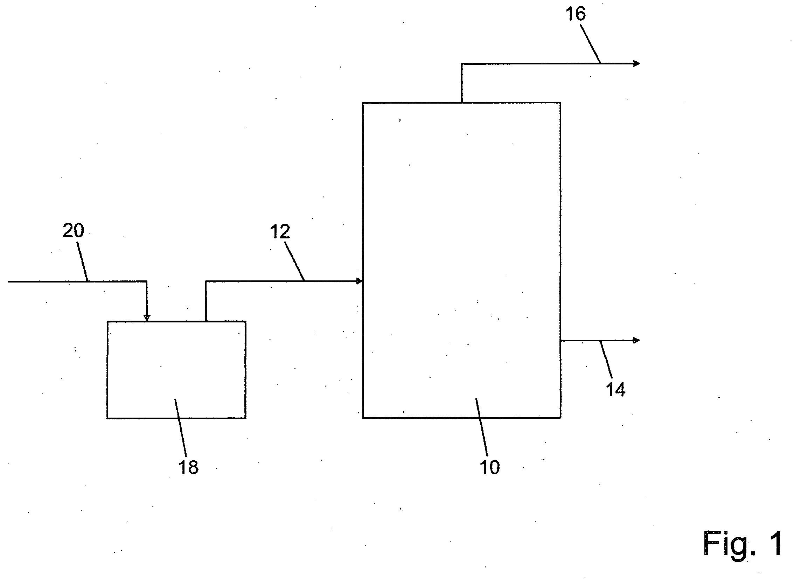

Figur 1 zeigt einen Ausschnitt aus dem Fließbild einer Biogasanlage. Ein Fermenter trägt das Bezugszeichen 10. Der Fermenter 10 verfügt über einen Zulauf 12 und einen Ablauf 14 und einen Abzug 16 für Biogas. Am Zulauf 12 des Fermenters 10 ist ein Behälter 18 angeordnet.

Die Funktionsweise des in Figur 1 dargestellten Ausschnitts ist folgendermaßen: organische Stoffe, wie beispielsweise Energiepflanzen (z.B. Mais und andere Getreide, Schilfgras), Reststoffe wie Ernterückständen (z.B. Rübenblätter), tierische Exkremente (z.B. Gülle, Mist), Nebenprodukte der Lebensmittelproduktion (z.B. Fette, Speisereste, Kartoffelschalen) oder organische Abfälle (z.B. Klärschlamm) werden, dargestellt durch Pfeil 20, in den Behälter 18 eingefüllt.

Die Befüllung des Behälters 18 mit festen Stoffen erfolgt durch einen Radlader, einen Frontlader, einen Teleskoplader oder ähnliche Baumaschinen oder landwirtschaftliche Maschinen. Denkbar ist auch die Befüllung des Behälters 18 mittels eines Förderbandes aus einem Dosierbehälter.

Im Behälter 18 erfolgt eine Vermischung der organischen Stoffe mit flüssigen Gärresten oder Gülle. Ein so angemischtes Substrat wird anschließend chargenweise oder bei kleinem Behältervolumen im Gesamten dem Fermenter 10 zugeführt. Im Fermenter 10 erfolgt der mikrobielle, anaerobe Abbau organischer Substanz. Dabei wird Biogas erzeugt, das über den Abzug 16 einem Blockheizkraftwerk zugeführt wird oder einer Nachbehandlung zugeführt wird, so dass es als Biomethan in ein Erdgasnetz eingespeist werden kann.

Ein trockener oder nasser Gärrest wird über den Ablauf 14 aus dem Fermenter 10 ausgetragen und im Wesentlichen einer Verwendung als Düngemittel zugeführt.

Der Behälter 18 ist in der Regel ein aus Beton gefertigter zylindrischer Hohlkörper, der über eine rechteckige Einwurföffnung verfügt. Zur Vermeidung von Geruchsbelästigung ist die Einwurföffnung erfindungsgemäß mit einem Schleusensystem 22 versehen.

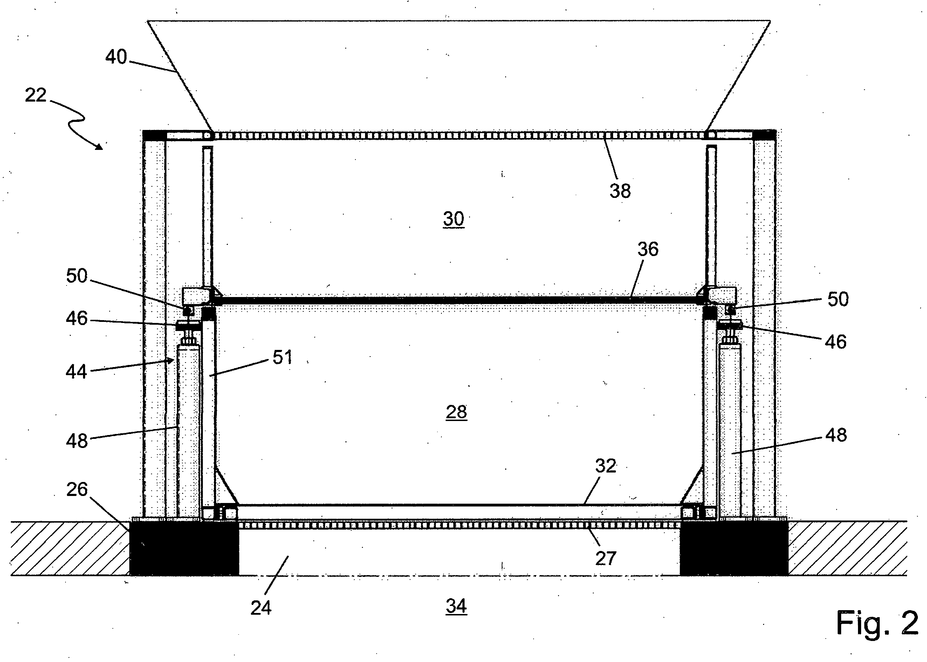

Das Schleusensystem 22 zeigt Figur 2 in einer Vorderansicht. Dargestellt ist ein Schnitt durch die rechteckige Einwurföffnung 24 in einer Decke 26 des Behälters 18. Die Einwurföffnung 24 ist mit einem ersten Gitterrost 27 ausgefüllt. Die Vorderansicht des Schleusensystems 22 erfolgt aus Richtung des anfahrenden Radladers.

Das Schleusensystem 22 umfasst eine erste Kammer 28 und eine zweite Kammer 30. Ein erster Schieber 32 ist zwischen der Einwurföffnung 24 und der ersten Kammer 28 angeordnet. Der erste Schieber 32 trennt in einer Geschlossenstellung die erste Kammer 28 von einem Inneren 34 des Behälters 18. Zwischen der ersten Kammer 28 und der zweiten Kammer 30 ist ein zweiter Schieber 36 angeordnet. Der zweite Schieber 36 trennt in einer Geschlossenstellung die erste Kammer 28 von der zweiten Kammer 30. Die zweite Kammer 30 ist nach oben durch einen zweiten Gitterrost 38 begrenzt. Die Abmessungen der Kammern 28, 30 sind an ein Volumen der Radladerschaufel angepasst. Der Gitterost 38 ist an einer in der Zeichnung linken, rechten und hinteren Seite, von jeweils einer Wand begrenzt. Die drei Wände sind beispielsweise aus Edelstahlblechen gefertigt und bilden eine Art Trichter 40, der zu einer Befüllseite 42, das heißt zum Radlader, hin offen ist. Eine leichte Neigung der Wände vom zweiten Gitterost 38 weg, ermöglicht eine Aufweitung einer Schleusenöffnung auf die Breite der Schaufel des Radladers. Somit rutschen die organischen Stoffe leicht und ohne Verlust auf den zweiten Gitterost 38 und in die zweite Kammer 30. Enthält das vom Radlader eingefüllte Substrat größerer Klumpen, wird es über die offene Befüllseite 42 des Trichters 40 leicht mit einer Schaufel des Radladers durch den zweiten Gitterrost 38 gedrückt. Der zweite Gitterost 38 ist insofern nicht erfindungswesentlich, als die Erfindung auch ohne ihn funktioniert. Der zweite Gitterrost 38 wird manchmal zur Unfallverhütung von den zuständigen Stellen gefordert. Die gleichen Grundsätze gelten auch für den ersten Gitterrost 27. Der Trichter 40 kann separat abgestützt werden, um Beschädigungen an einer, nachfolgend näher erläuterten, Wiegeeinrichtung 44 zu vermeiden.

Hat das Substrat den zweiten Gitterrost 38 passiert, fällt es auf den geschlossenen zweiten Schieber 36. Der zweite Schieber 36 dient in einer Geschlossenstellung als Wiegeeinrichtung 44. Die Wiegeeinrichtung 44 umfasst vier am Umfang des zweiten Schiebers 36 angeordnete Wiegezellen 50 sowie vier Wiegeplatten 46. Die Wiegeplatten 46 sind auf vier separaten Stützen 48 angeordnet; alternativ kann der Rahmen 51 der unteren Kammer 28 so stark ausgeführt werden, dass auf ihr die Wiegebolzen angebracht werden können. Die vier Wiegezellen 50 sind fest in die Wiegeplatten 46 eingebaut.

Um Wiegefehler zu vermeiden, ist es erforderlich, dass kein Kontakt zwischen einem Rahmen 51 der ersten Kammer 28 und dem zweiten Schieber 36 besteht beziehungsweise, dass der Rahmen 51 keine Stützfunktion auf die zweite Kammer 30 ausübt. Damit wird das auf dem zweiten Schieber 36 aufliegende Substrat gewogen und an eine Anlagensteuerung (nicht dargestellt) übermittelt.

Ein Fahrer des Radladers kann das eingefüllte Gewicht, die Bezeichnung der Komponenten (z.B. Mais, Mist, ....), das aufaddierte Gewicht der jeweiligen Komponenten, das Gesamtgewicht, die Position beider Schieber und den Behälterinhalt an einem großen mehrzeiligen Display ablesen. Wurde vom Fahrer vor Einfüllen die Art der organischen Substanz (beispielsweise Silomais, Gassilage, Festmist oder Ähnliches) an eine Wiegesoftware übermittelt, kann diese das eingefüllte Gewicht je Komponente aufaddieren. Ist ein gewünschtes Gewicht der jeweiligen Komponente nach Einfüllen mehrerer Schaufeln erreicht, drückt der Fahrer die nächste Komponententaste auf seiner Fernbedienung und fährt mit der Befüllung mit der nächsten Komponente fort. Es ist auch denkbar, dass die Anlagensteuerung automatisch zur nächsten Komponente wechselt, wenn ein vorgebbares Gewicht erreicht ist. Ist das geforderte Gewicht einer letzten Komponente erreicht, wird das Programm beendet. Dies kann auch durch Drücken der "Ende"-Taste auf der Fernbedienung erfolgen. Die Wiegesoftware führt eine Statistik über die Einfüllgewichte jeder Komponente. Dies kann dann beispielsweise monatlich an einen Umweltbeauftragten geschickt werden, der die Biogasanlage kontrolliert.

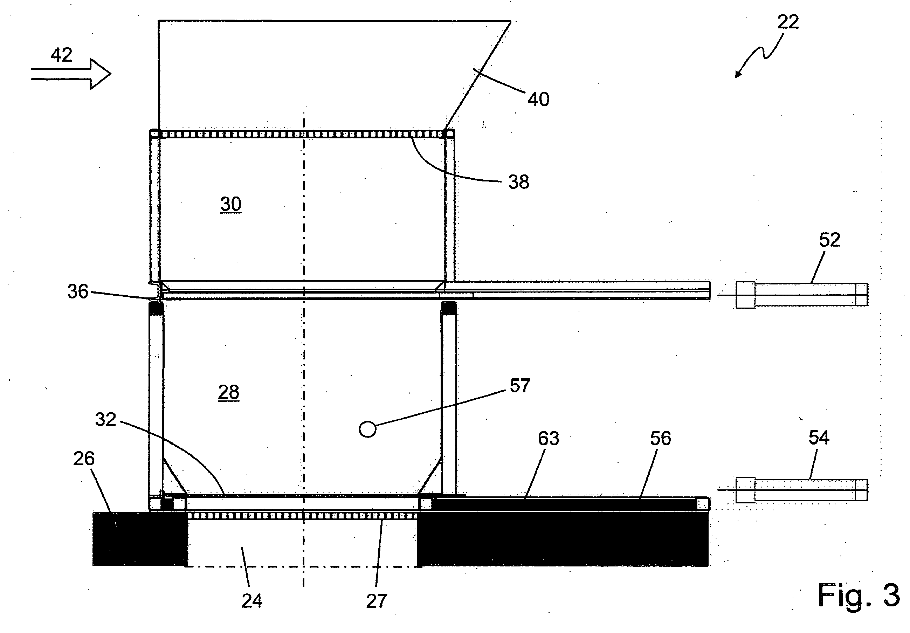

Wenn sich das Gewicht des Substrats, das sich auf dem zweiten Schieber 36 befindet, stabilisiert hat, wird der zweite Schieber 36 durch einen ersten Hydraulikzylinder 52 (dargestellt in Figur 3) in eine Offenstellung bewegt, und das Substrat fällt in die darunterliegende erste Kammer 28. Das Substrat kommt in der ersten Kammer 28 auf dem geschlossenen ersten Schieber 32 zu liegen. Daraufhin wird der zweite Schieber 36 wieder geschlossen. Erst wenn der zweite Schieber 36 geschlossen ist, wird der erste Schieber 32 durch einen zweiten Hydraulikzylinder 54 geöffnet und das Substrat fällt durch den ersten Gitterrost 27 in das Innere 34 des Behälters 18.

Außer zu Revisionszwecken sind niemals beide Schieber 32, 36 gleichzeitig geöffnet. Um trotzdem die beiden Kammern 28, 30 zugänglich zu machen, verfügen die Kammern 28, 30 über jeweils ein Mannloch 55, das in einer Wand der Kammern 28, 30 angeordnet ist.

Figur 3 zeigt das Schleusensystem 22 in einer Seitenansicht. Die Befüllseite 42 befindet sich in der Zeichnung links. Das heißt der Radlader fährt von links an und kippt das Substrat mit der Schaufel in den links offenen Trichter 40. Das Substrat rutscht an den schrägen Wänden des Trichters 40 auf den zweiten Gitterrost 38 und fällt in die zweite Kammer 30 auf den geschlossenen zweiten Schieber 36. Es ist denkbar, den zweiten Schieber 36 gegenüber der ersten Kammer 28 mit einer Sperrflüssigkeit abzudichten, eine einfachere Lösung ist es jedoch, eine Gummiabdichtung des zweiten Schiebers 36. Ist nach Einfüllen einer oder mehrerer Schaufelladungen ein gewünschtes Gewicht erreicht, wird der, als Wiegeplatte ausgeführte, zweite Schieber 36 vom ersten Hydraulikzylinder 52 nach hinten, in Figur 3 rechts, gezogen.

Die Führung des zweiten Schiebers 36 kann mittels Gleitschienen (nicht dargestellt) oder ähnlichem erfolgen. Damit der zweite Schieber 36 auch bei großen Temperaturdifferenzen zwischen Behälterinnerem 34 und Umgebung, wie sie beispielsweise im Winter auftreten, sicher öffnet und schließt ist, ist optional eine Heizeinrichtung vorgesehen.

Öffnet der zweite Schieber 36 fällt das darauf liegende Substrat nach unten, in die erste Kammer 28, wo es auf dem geschlossenen ersten Schieber 32 liegen bleibt.

Eine Abdichtung des geschlossenen ersten Schiebers 32 gegenüber dem Behälterinneren 34 erfolgt mittels einer Sperrflüssigkeit, bevorzugt Wasser. Dazu ist unterhalb des ersten Schiebers 32 in Offenstellung ein abgedecktes Becken 56 angeordnet. Das Becken 56 ist in seinen Abmessungen so gestaltet, dass der geöffnete erste Schieber 32 es gerade überdeckt.

Gerüche, die bei geöffnetem ersten Schieber 32 aus dem Behälterinneren 34 in die erste Kammer 28 gelangen, werden durch eine Öffnung 57 in einen Biofilter (nicht dargestellt) optional abgesaugt.

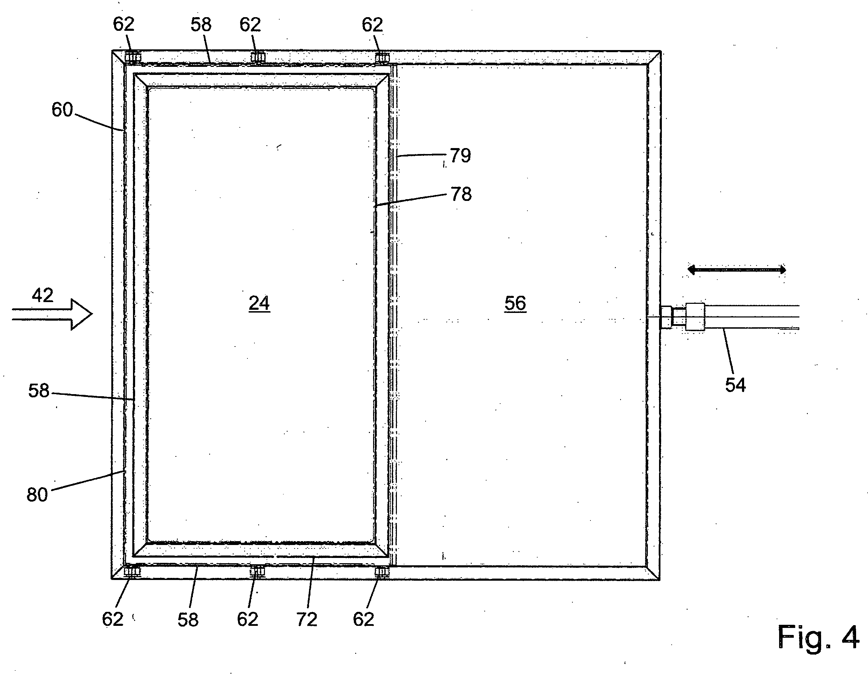

Figur 4 zeigt in einer Draufsicht die Einfüllöffnung 24. Zur Orientierung ist in der Zeichnung links ein Pfeil angeordnet, der die Befüllseite 42 markiert. In der Zeichnung rechts ist der zweite Hydraulikzylinder 54 skizziert.

Damit Sperrflüssigkeit die Einfüllöffnung 24 vollständig umgibt, sind an drei Seiten der Einfüllöffnung 24, miteinander verbundene abgedeckte Rinnen 58 angeordnet. Die Rinnen 58 stehen mit dem Becken 56 in hydraulischer Verbindung. Dadurch ist sichergestellt, dass sich im Becken 56 und in den drei Rinnen 58 dasselbe Flüssigkeitsniveau einstellt. Das Flüssigkeitsniveau wird durch eine Sonde (nicht dargestellt) überwacht. Weiterhin verfügt das Becken 56 über eine Heizeinrichtung (nicht dargestellt), die die Sperrflüssigkeit vor Einfrieren schützt.

Der in Figur 4 durch eine strichpunktierte Linie 60 angedeutete erste Schieber 32 überdeckt in der Geschlossenstellung die drei Rinnen 58 sowie eine der Befüllseite 42 nächstgelegen Teil des Wasserbeckens 56.
In seiner Offenstellung überdeckt der erste Schieber 32 das Becken 56 vollständig. Oberhalb des Beckens 56 und des ersten Schiebers 32 ist an einer, der Befüllseite 42 abgewandten, Längsseite 59 des ersten Schiebers 32, ein Abstreifer 61 angeordnet. Der Abstreifer 61 überdeckt die gesamte Längsseite 59 des ersten Schiebers 32 und dient dazu, eventuell auf dem ersten Schieber 32 aufliegende organische Stoffe beim Öffnen des ersten Schiebers 32 in die Einwurföffnung 24 abzustreifen.

Seitlich am ersten Schieber 32, in der Zeichnung oben und unten, sind mehrere Laufrollen 62 angeordnet. Die Laufrollen 62 wälzen neben der seitlichen Rinnen 58 ab und gewährleisten so das sichere Öffnen und Schließen des ersten Schiebers 32.

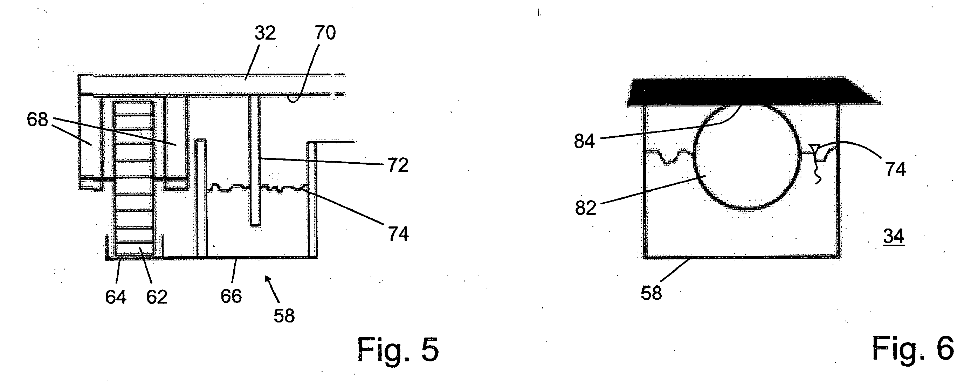

Eine Detailansicht einer Laufrolle 62 zeigt Figur 5. Dargestellt ist ein Schnitt durch eine seitliche Rinne 58. Die Rinne 58 wird vom geschlossenen ersten Schieber 32 überdeckt. Eine oberhalb der Rinne 58 angeordnete Abdeckung 63 schützt die in der Rinne 58 stehende Sperrflüssigkeit bei geschlossenem erstem Schieber 32, gegen Verschmutzung, und dichtet das gesamte Becken 56 nach oben ab.

Die Laufrolle 62 ist neben der Rinne in einer Schiene, die einen U-förmigen Querschnitt haben kann, angeordnet, so dass eine Lauffläche 64 über die Schiene abrollt. Gelagert ist die Laufrolle 62 dabei an einer Lasche 68, die an einer Unterseite 70 des ersten Schiebers 32 angeordnet ist.

In der Zeichnung rechts von der Laufrolle 62 ragt ein Steg 72 von oben in die Rinne 58. Der Steg 72 ist von unten am ersten Schieber 32 durch schweißen, kleben, nieten oder ähnliche Methoden gasdicht befestigt. Eine zum ersten Schieber 32 senkrechte Ausdehnung des Stegs 72 ist so bemessen, dass er bis unter das Flüssigkeitsniveau 74 in der Rinne 58 ragt, aber nicht am Rinnenboden 66 anliegt. In diesem Beispiel ist der Querschnitt der Rinne 58 100 mm breit und 120 mm hoch, die Ausdehnung des Stegs senkrecht zum ersten Schieber 32 beträgt 130 mm.

In horizontaler Richtung erstreckt sich der Steg 72 über die gesamte Länge des ersten Schiebers 32.

In Zeichnung 4 sind also die obere und die untere Seite des ersten Schiebers 32 mit einem Steg 72 versehen. Ebenso ist eine dritte, der Befüllseite 42 abgewandte Seite 76 des ersten Schiebers mit einem dritten Steg 78 versehen. Die seitlichen Stege 72 sind mit dem dritten Steg 78 an ihren Enden gasdicht verbunden. Auf diese Weise sind drei Seiten des ersten Schiebers 32 gegen das Behälterinnere 34 abgedichtet. Ein Ausführungsbeispiel einer Abdichtung einer an der Befüllseite 42 des Schleusensystems angeordneten Stirnseite 80 des ersten Schiebers 32 ist in Figur 6 dargestellt.

Figur 6 zeigt im Querschnitt die Rinne 58 die parallel zur Befüllseite 42 angeordnet ist. In der Rinne 58 angeordnet ist ein Schwimmkörper 82. Der Schwimmkörper 82 verfügt über einen beispielsweise kreisförmigen Querschnitt. Es sind aber auch andere Querschnittformen denkbar. Seine Ausdehnung längs der Rinne 58 ist so bemessen, dass er bei geschlossenen ersten Schieber 32 mit beiden Enden an den Stegen 72 anliegt. Der Schwimmkörper 82 schwimmt so auf der Flüssigkeit in der Rinne 58, dass ein oberster Punkt so weit über das Flüssigkeitsniveau 74 ragt, dass der Schwimmkörper 80 vom schließenden, ersten Schieber 32 in die Flüssigkeit gedrückt wird. Bei geschlossenem, erstem Schieber 32, drückt die Auftriebskraft den Schwimmkörper 82 mit dem obersten Punkt 84 gegen die Unterseite 70 des ersten Schiebers 32 und dichtet ihn damit gegen das Behälterinnere 34 ab.

Eine einfachere Möglichkeit (nicht dargestellt) die Stirnseite des ersten Schiebers abzudichten, stellt die Anordnung einer großvolumigen Gummidichtung zwischen Stirnseite 80 und Befüllseite 42 dar. Eine Dichtwirkung wird dabei durch den Anpressdruck erzielt, mit dem der zweite Hydraulikzylinder 54 den ersten Schieber gegen die Gummidichtung presst.

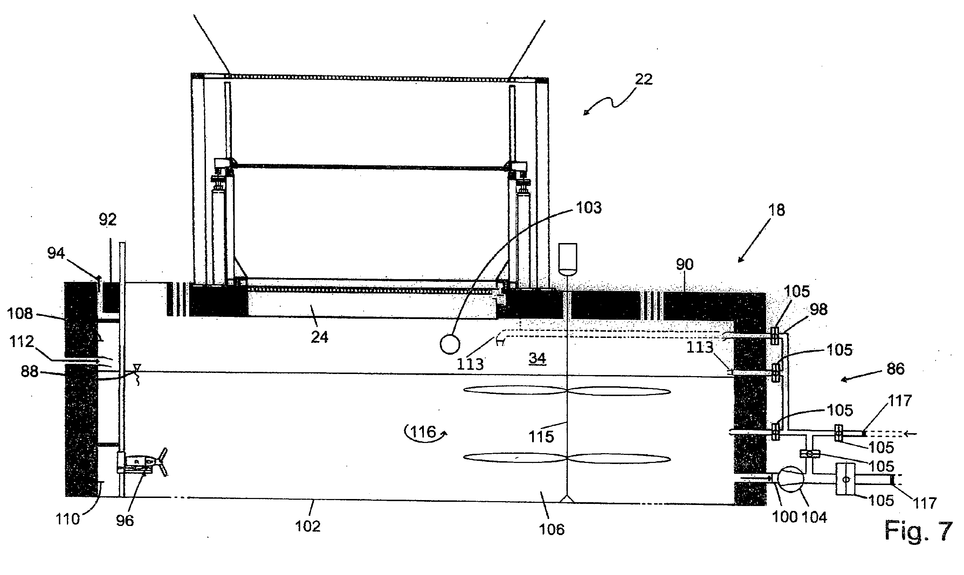

Figur 7 zeigt beispielsweise einen Anmaischbehälter 86, ausgestattet mit dem Schleusensystem 22. Der dargestellte Anmaischbehälter 86 wird in der Regel als zylinderförmiger Hohlkörper ausgebildet, der in beispielsweise über eine Durchmesser von 4 m und eine Tiefe von 2 m verfügt. Mit einem Fassungsvermögen von 12,5 m³ liegt ein Füllstand 88 des Anmaischbehälter bei ca. halber Tiefe.

In einer Behälterdecke 90 sind die Einwurföffnung 24 eine Revisionsöffnung 92 und eine Luftabsaugung 94 angeordnet. Über die Luftabsaugung werden im Behälterinneren entstehende Gerüche mittels eins Gebläses abgesaugt und stark mit Umgebungsluft verdünnt oder in einem nachgeordneten Biofilter zersetzt.

Durch die Revisionsöffnung 92 wird ein im Behälter 18 angeordnetes höhenverstellbares Rührwerk 96 gewartet. Alternativ oder ergänzend zum Rührwerk 96 ist eine Umpumpleitung 98 vorgesehen. Die Umpumpleitung zweigt von einer Entnahmeleitung 100 ab. Die Entnahmeleitung 100 ist etwa 0,5 m oberhalb eines Bodens 102 des Behälters 18 angeordnet. In der Entnahmeleitung 100 ist eine Entnahmepumpe 104 angeordnet. Die Entnahmepumpe 104 saugt das Substrat aus dem Behälterinneren 34 an und fördert es entweder weiter in den Fermenter 10 oder über die Umpumpleitung 98 zurück in den Behälter 18. Somit wird eine Durchmischung des Substrates erreicht.

Die Umpumpleitung kann auch noch verlängert werden, bis unter die Einwurfkante der Schleuse und am Ende mit einer schräg nach unten, am Besten in Drehrichtung des Behälters ausgerichteten, Düse 113 ausgerüstet werden. Durch die Befeuchtung der frisch eingeworfenen Feststoffe kann die Einrührleistung des parallel arbeitenden Rührsystems 96 und115 deutlich gesteigert werden. Das Rührsystem 115 kann mit bevorzugt horizontalen Flügeln 115, die in die Einwurfsöffnung 24 hineinreichen können, ausgerüstet sein.

Die Düse 113 kann auch wahlweise oder zusätzlich seitlich an der Behälterwand im Bereich des Feststoffeinwurfes möglichst schräg in Drehrichtung 116 des Behälters angebracht werden. Es ist auch möglich die Düse 113 in einer im Sprühwinkel verstellbaren Ausführung einzubauen und mit wechselbaren verschiedengroßen Sprühköpfen auszurüsten. Die Verdünnungsflüssigkeitszufuhr kann auch aus einem anderen Behälter, z.B Endlagerbehälter, über eine extra Pumpe erfolgen. Die Menge wird über einen Durchflusszähler 117 gesteuert. Der Startzeitpunkt kann mit dem Wiegesystem der Schleuse gekoppelt werden und erst nach Einwurf einiger Tonnen Feststoffe, wenn sich die Anmaischflüssigkeit beginnt zu verdicken, erfolgen. Möglich ist auch, dass bei jeder Öffnung des ersten Schiebers 32 eine bestimmte Menge an Verdünnungsflüssigkeit automatisch zugeführt wird.

Um die Durchmischung des Substrates zu überwachen, ist in einer Behälterwand ein Schauglas 103 angeordnet. Das Schauglas kann mit einer Kamera bestückt sein, die Bilder vom Behälterinneren 34 an einen Bildschirm überträgt.

Eine Umschaltung zwischen Entnahmeleitung 100 und Umpumpleitung 98 erfolgt mittels automatischer Absperrarmaturen 105. In der Zeichnung dargestellt sind Scheibenventile, denkbar sind aber auch Schieber oder ein als 4/2-Wegeventil ausgeführtes Sitzventil.

Durch eine Anordnung der Entnahmeleitung 100 etwas oberhalb des Behälterbodens 102 ist sichergestellt, dass der Behälter 18 nicht vollständig entleert wird und damit Fremdkörper, wie beispielsweise Steine, die nachfolgende Anlagenkomponenten oder Apparate schädigen können in einem Behältersumpf 106 zurückgehalten werden.

Aus sicherheitstechnischen Gründen ist in einem oberen Behälterbereich ist eine erste Sonde 108 angeordnet, die eine Überfüllung des Behälters 18 detektiert. Optional kann in einem unteren Behälterbereich eine zweite Sonde 110 angeordnet sein, die das Erreichen eines minimalen Füllstandes detektiert, bzw. den aktuellen Füllstand an die Steuerung meldet.

Der dargestellte Anmaischbehälter 86 funktioniert folgendermaßen: Während der Radlader die einzufüllenden organischen Stoffe holt, wird der Anmaischbehälter 86 bereits bis zum Füllstand 88, also bis ca. zur Hälfte, mit Flüssigkeit aus Gärresten oder Frischgülle befüllt. Die Befüllung erfolgt über eine separate Leitung 112 und wird mit einem Durchflusszähler überwacht. Es versteht sich, dass für die Befüllung eine leistungsstarke Pumpe eingesetzt wird, damit lange Wartezeiten für den Radladerfahrer vermieden werden.

Bevor das Schleusensystem 22 durch den Radlader beschickt wird, sind das Rührwerk 96 bzw. 115 und/oder die Entnahmepumpe mit geöffneter Umpumpleitung 98 in Betrieb. Die organischen Substanzen fallen über das Schleusensystem, wie bereits voranstehend beschrieben, in den Anmaischbehälter 86 und werden durch die Rührwerke 94/115 und/oder Umpumpen des Behälterinhaltes mit der Flüssigkeit vermischt. Ob das Rührwerk 96 benötigt wird, hängt ab von der Art der organischen Stoffe die eingebracht werden oder von einer gewünschten Zeit, die das Vermischen der organischen Substanzen mit Flüssigkeit benötigt.

Eine Entnahme des Substrats aus dem Anmaischbehälter 86 erfolgt über die Entnahmepumpe 104. Ein Starten der Entnahmepumpe 104 und ein Schließen der Umpumpleitung 98 bzw. Öffnen der Entnahmeleitung 100 erfolgt über einen Durchflusszähler 117 bzw. einer Zeitschaltuhr, die nach einem Erfahrungswert eingestellt wird. So kann beispielsweise die Entnahmepumpe 104 3 min nach Schließen des ersten Schiebers 32 anlaufen.

Bei einem kleinen Anmaischbehälter 86 wird der gesamte Inhalt, mit Ausnahme des Behältersumpfs 106, in einem Schritt dem Fermenter 10 zugeführt.

Je nach Art der organischen Stoffe bzw. der Größe der Schaufel des Radladers, ist es möglich, mehrere Schaufeln über das Schleusensystem 22 dem Anmaischbehälter 86 zuzuführen bevor eine Entnahme erfolgt. Dazu kann die Entnahmepumpe 104 beispielsweise per Fernbedienung, manuell oder nach eine vorbestimmbaren Anzahl an Öffnungen des ersten Schiebers 32 gestartet werden.

Im Unterschied zum Anmaischbehälter 86 verbleibt bei einem größeren Hydrolysebehälter das Substrat längere Zeit im Behälter 18 und wird dann in regelmäßigen Abständen, beispielsweise alle zwei Stunden, in den Fermenter 10 gefördert. Hydrolysebehälter verfügen beispielsweise über ein Füllvolumen von 200 m³. Hierbei wird auch zumeist mehr als ein Rührwerk benötigt.

Die Anzahl der Rührwerke ist abhängig von der Behältergröße und den eingesetzten Futterkomponenten.

Durch regelmäßige z.B. tägliche Zufuhr der Futterkomponenten und Durchflusszähler 117 gesteuertes Zupumpen von Verdünnungsflüssigkeit z.B. aus einem Endlagerbehälter, kann ein gleichbleibender Mindestfüllstand von ca. 50%-90% in dem Behälter 18 gehalten werden. Dies ermöglicht die Versäuerung des Substrates bei einem ph-Wert zwischen 4,5 bis 7.

## Patentansprüche

1. Behälter (18) zur Vorbehandlung von organischen Stoffe, angeordnet in einer Biogasanlage, wobei die Befüllung des Behälters (18) mit organischen Stoffen über ein Schleusensystem (22) erfolgt, **dadurch gekennzeichnet, dass** das Schleusensystem (22) eine erste Kammer (28) und eine zweite Kammer (30)umfasst, wobei die erste Kammer (28) durch einen ersten Schieber (32) von einer Einfüllöffnung (24) des Behälters (18) abtrennbar ist, und wobei die zweite Kammer (30) durch einen zweiten Schieber (36) von der ersten Kammer (28) abtrennbar ist.

2. Behälter (18) nach einem der vorangehenden Ansprüche, **dadurch gekennzeichnet, dass** die Schieber (32, 36) hydraulisch betätigt sind.

3. Behälter (18) nach einem der vorangehenden Ansprüche, **dadurch gekennzeichnet, dass** die Schieber (32, 36) in Schienen geführt sind.

4. Behälter (18) nach einem der vorangehenden Ansprüche, **dadurch gekennzeichnet, dass** der zweite Schieber (36) mit einer Wiegeeinrichtung (44) verbunden ist, und dass die Wiegeeinrichtung (44) bevorzugt mit einem großen Display und/oder einer Funkfernbedienung ausgerüstet ist.

5. Behälter (18) nach einem der vorangehenden Ansprüche, **dadurch gekennzeichnet, dass** er einen Trichter (40) aufweist, wobei der Trichter (40) zu einer Befüllseite (42) offen ist.

6. Behälter (18) nach einem der vorangehenden Ansprüche, **dadurch gekennzeichnet, dass** mindestens ein Schieber (32, 36) an mindestens einer Seite einen Steg (72) aufweist, der sich über die gesamte Länge der Seite erstreckt und der von oben in eine flüssigkeitsgefüllte Rinne (58) oder ein flüssigkeitsgefülltes Becken (56) taucht.

7. Behälter (18) nach einem der vorangehenden Ansprüche, **dadurch gekennzeichnet, dass** mindestens ein Schieber (32, 36) an allen Seiten eine Gummidichtung aufweist.

8. Behälter (18) nach einem der vorangehenden Ansprüche, **dadurch gekennzeichnet, dass** eine Stirnseite (80) des Schiebers (32, 36) eine Gummidichtung aufweist.

9. Behälter (18) nach einem der vorangehenden Ansprüche, **dadurch gekennzeichnet, dass** das Becken (56) eine Heizeinrichtung und eine Füllstandsüberwachung umfasst und mit den flüssigkeitsgefüllten Rinne (58) in hydraulischer Verbindung steht, wobei die Rinnen (58) und das Becken (56) über eine Abdeckung (63) verfügen.

10. Behälter (18) nach einem der vorangehenden Ansprüche, **dadurch gekennzeichnet, dass** das Becken (56) unterhalb des Schiebers (32,36) in Offenstellung angeordnet ist.

11. Behälter (18) nach einem der vorangehenden Ansprüche, **dadurch gekennzeichnet, dass** oberhalb einer Längsseite (59) des Schiebers (32,36) ein Abstreifer (79) angeordnet ist.

12. Behälter (18) nach einem der vorangehenden Ansprüche, , **dadurch gekennzeichnet, dass** er ein höhenverstellbares Rührwerk (96) und/oder mindestens ein starr eingebautes Rührwerk (115) mit mehreren waagerechten Flügeln umfasst, und dass eine von den waagerechten Flügeln überstrichene Fläche sich teilweise unterhalb der Einfüllöffnung (24) befindet.

13. Behälter (18) nach einem der vorangehenden Ansprüche, **dadurch gekennzeichnet, dass** er eine Entnahmepumpe (104), eine Entnahmeleitung (100) und eine Umpumpleitung (98) umfasst, wobei die Umpumpleitung (98) durch ein Absperrorgan (105) trennbar mit der Entnahmeleitung (100) verbunden ist.

14. Behälter (18) nach einem der vorangehenden Ansprüche **dadurch gekennzeichnet, dass** es sich um einem Hydrolysebehälter handelt.

15. Behälter nach einem der vorangehenden Ansprüche **dadurch gekennzeichnet, dass** er mit einem optional verstellbaren Düsensystem ausgerüstet ist, das die frisch eingeworfenen Feststoffe mit Flüssigkeit besprüht um ein schnelleres Einrühren zu ermöglichen; und dass die Düsen (113) bevorzugt in Drehrichtung der Flüssigkeit im Behälter schräg eingebaut bzw. eingestellt sind, und dass die Düsen (113) in die Flüssigkeit eintauchen können.

## Claims

1. A container (18) for pretreating organic substances, located in a biogas system, in which the filling of the container (18) with organic substances is effected via a sluice system (22), **characterized in that** the sluice system (22) includes a first chamber (28) and a second chamber (30), and the first chamber (28) can be disconnected from an infeed opening (24) of the container by means of a first slide (32), and the second chamber (30) can be disconnected from the first chamber (28) by means of a second slide (36).

2. The container (18) of the foregoing claim, **characterized in that** the slides (32, 36) are hydraulically actuated.

3. The container (18) of one of the foregoing claims, **characterized in that** the slides (32, 36) are guided in rails.

4. The container (18) of one of the foregoing claims, **characterized in that** the second slide (36) communicates with a weighing device (44), and that the weighing device (44) is preferably equipped with a large display and/or a radio remote control.

5. The container (18) of one of the foregoing claims, **characterized in that** it has a funnel (40), and the funnel (40) is open toward an input side (42).

6. The container (18) of one of the foregoing claims, **characterized in that** at least one slide (32, 36), on at least one side, has a frame link (72), which extends over the full length of that side and which plunges from above into a liquid-filled channel (58) or a liquid-filled basin (56).

7. The container (18) of one of the foregoing claims, **characterized in that** at least one slide (32, 36) has a rubber seal on all sides.

8. The container (18) of one of the foregoing claims, **characterized in that** one face end (30) of the slide (32, 36) has a rubber seal.

9. The container (18) of one of the foregoing claims, **characterized in that** the basin (56) includes a heating device and a fill level monitor and communicates hydraulically with the liquid-filled channel (58), and the channel (58) and the basin (56) have a covering (63).

10. The container (18) of one of the foregoing claims, **characterized in that** the basin (56) is located below the slide (32, 36) in the open position.

11. The container (18) of one of the foregoing claims, **characterized in that** a squeegee (79) is located above one long side (59) of the slide (32, 36).

12. The container (18) of one of the foregoing claims, **characterized in that** it includes an adjustable-height agitator mechanism (96) and/or at least one rigidly built-in agitator mechanism (115) having a plurality of horizontal vanes, and that a surface swept over by the horizontal vanes is located partly below the feed opening (24).

13. The container (18) of one of the foregoing claims, **characterized in that** it includes a discharge pump (104), a discharge line (100), and a transfer pumping line (98), and the transfer pumping line (98) is connected disconnectably, by a blocking device (105), to the discharge line (100).

14. The container (18) of one of the foregoing claims, **characterized in that** it is a hydrolysis container.

15. The container (18) of one of the foregoing claims, **characterized in that** it is equipped with an optionally adjustable nozzle system, which sprays the newly thrown-in solids with liquid in order to make it possible to stir them in faster; and that the nozzles (113) are preferably built in or adjusted obliquely in the direction of rotation of the liquid in the container, and that the nozzles (113) are capable of plunging into the liquid.

## Revendications

1. Contenant (18) destiné au prétraitement de matières organiques, disposé dans une installation de biogaz, le remplissage du contenant (18) de matières organiques se faisant par le biais d'un système d'écluse (22), **caractérisé par le fait que** ledit système d'écluse (22) comprend une première chambre (28) et une deuxième chambre (30), dans lequel ladite première chambre (28) peut être séparée d'une ouverture de remplissage (24) du contenant (18) par l'intermédiaire d'un premier tiroir (32), et dans lequel ladite deuxième chambre (30) peut être séparée de la première chambre (28) par l'intermédiaire d'un deuxième tiroir (36).

2. Contenant (18) selon la revendication précédente, **caractérisé par le fait que** lesdits tiroirs (32, 36) sont actionnés de façon hydraulique.

3. Contenant (18) selon l'une quelconque des revendications précédentes, **caractérisé par le fait que** lesdits tiroirs (32, 36) sont guidés dans des rails.

4. Contenant (18) selon l'une quelconque des revendications précédentes, **caractérisé par le fait que** le deuxième tiroir (36) est relié à un dispositif de pesée (44) et que ledit dispositif de pesée (44) est équipé de préférence d'un grand écran de visualisation et/ou d'une télécommande radio.

5. Contenant (18) selon l'une quelconque des revendications précédentes, **caractérisé par le fait qu'**il présente une trémie (40), ladite trémie (40) étant ouverte vers un côté de remplissage (42).

6. Contenant (18) selon l'une quelconque des revendications précédentes, **caractérisé par le fait qu'**au moins un tiroir (32, 36) présente, sur au moins un côté, une entretoise (72) qui s'étend sur l'ensemble de la longueur du côté et qui plonge d'en haut dans une rigole (58) remplie de liquide ou dans un bassin (56) rempli de liquide.

7. Contenant (18) selon l'une quelconque des revendications précédentes, **caractérisé par le fait qu'**au moins un tiroir (32, 36) présente un joint d'étanchéité en caoutchouc sur tous les côtés.

8. Contenant (18) selon l'une quelconque des revendications précédentes, **caractérisé par le fait qu'**un côté frontal (80) du tiroir (32, 36) présente un joint d'étanchéité en caoutchouc.

9. Contenant (18) selon l'une quelconque des revendications précédentes, **caractérisé par le fait que** ledit bassin (56) comprend un dispositif de chauffage et un contrôle de niveau et est en communication hydraulique avec la rigole (58) remplies de liquide, la rigole (58) et le bassin (56) disposant d'une couverture (63).

10. Contenant (18) selon l'une quelconque des revendications précédentes, **caractérisé par le fait que** le bassin (56) est disposé au-dessous du tiroir (32, 36) en position ouverte.

11. Contenant (18) selon l'une quelconque des revendications précédentes, **caractérisé par le fait qu'**un racleur (79) est disposé au-dessus d'un grand côté (59) du tiroir (32, 36).

12. Contenant (18) selon l'une quelconque des revendications précédentes, **caractérisé par le fait qu'**il comprend un agitateur (96) réglable en hauteur et/ou au moins un agitateur (115) monté de manière rigide et ayant une pluralité de pales horizontales, et qu'une surface balayée par lesdites pales horizontales est située en partie au-dessous de ladite ouverture de remplissage (24).

13. Contenant (18) selon l'une quelconque des revendications précédentes, **caractérisé par le fait qu'**il comprend une pompe de prise (104), une conduite de prise (100) et une conduite de recirculation (98), ladite conduite de recirculation (98) est reliée de manière séparable, par un organe d'arrêt (105), à ladite conduite de prise (100).

14. Contenant (18) selon l'une quelconque des revendications précédentes, **caractérisé par le fait qu'**il s'agit d'un réservoir hydrolytique.

15. Contenant (18) selon l'une quelconque des revendications précédentes, **caractérisé par le fait qu'**il est équipé d'un système à buses réglable de façon optionnelle qui pulvérise de liquide les matières solides fraîchement introduites afin de permettre de délayer plus rapidement ; et que, de préférence, les buses (113) sont montées ou bien réglées obliquement dans le sens de rotation du liquide dans le contenant, et que les buses (113) peuvent plonger dans le liquide.
